# EUROPÄISCHE PATENTSCHRIFT

(11) **EP 3 038 995 B1**
(45) Veröffentlichungstag und Bekanntmachung des Hinweises auf die Patenterteilung: **10.07.2019**
(21) Anmeldenummer: 13826735.6
(22) Anmeldetag: 20.12.2013
(51) Int. Cl.: C05C 9/00, C05D 5/00, C05D 9/00

(54) **ZUSAMMENSETZUNG EINER MAGNESIUMSULFAT-HARNSTOFF-VERBINDUNG**
COMPOSITION OF A MAGNESIUM SULPHATE-UREA COMPOUND
COMPOSITION D'UN COMPOSÉ SULFATE DE MAGNÉSIUM-URÉE

(30) Priorität: 21.12.2012 DE 102012025141
(43) Veröffentlichungstag der Anmeldung: 06.07.2016
(73) Patentinhaber: K+S Aktiengesellschaft, 34131 Kassel (DE)
(72) Erfinder: DIETRICH, Armin, 37299 Weißenborn (DE); BAUCKE, Guido, 36277 Schenklengsfeld (DE); THENERT, Stefan, 36266 Heringen (DE); KLEINE-KLEFFMANN, Ulrich, 36251 Bad Hersfeld (DE); WALCZYK, Wolfgang, 36266 Heringen (DE); DRESSEL, Stefan, 34128 Kassel (DE); WALDMANN, Ludger, 48291 Telgte (DE)
(74) Vertreter: Reitstötter Kinzebach
(86) Internationale Anmeldenummer: PCT/EP2013/077724
(87) Internationale Veröffentlichungsnummer: WO 2014/096372

(56) Entgegenhaltungen:
- WO-A1-2013/098367

## Beschreibung

Die vorliegende Erfindung betrifft Zusammensetzungen einer Magnesiumsulfat-Harnstoff-Verbindung der Formel (I) und von Gemischen der Verbindung der Formel (I) mit der Verbindung der Formel (II):

[MgSO₄ • m CO(NH₂)₂ • n H₂O] (I),

[MgSO₄ • x CO(NH₂)₂ • y H₂O] (II),

worin m und x im Bereich von 0,9 bis 1,1, n im Bereich von 1,9 bis < 2,1 und y im Bereich von 2,9 bis 3,1 liegen, die Herstellung der Zusammensetzungen und deren Verwendung als Düngemittel oder Düngemittelzusatz.

Obwohl Magnesium als achthäufigstes Element zu etwa 1,94 % in der Erdkruste vorhanden ist, weisen Böden oft einen Mangel an Magnesium auf. Deshalb finden Magnesiumsalze als Düngemittel oder Düngemittelzusätze eine breite Verwendung. Insbesondere werden Salze des Magnesiums als Düngemittel oder Düngemittelzusätze eingesetzt. Üblicherweise werden diese Salze als Magnesiumsulfat in Verbindung mit Makronährstoffen wie Kalium, Phosphor oder Stickstoff sowie mit Spurenelementen wie Mangan, Zink, Kupfer, Eisen, Kobalt, Molybdän oder Bor eingesetzt.

Grundsätzlich ist es von Interesse, möglichst viele Makro- und Mikronährstoffe gemeinsam in einer Düngemittelzusammensetzung bereitzustellen. Jedoch sind dem gemeinsamen Einsatz von Magnesium und Stickstoff Grenzen gesetzt. So ist eine einfache Feststoffmischung von Bittersalz (MgSO₄ • 7 H₂O) und Harnstoff nicht lagerstabil. Es kommt schon nach kurzer Zeit zu einer Reaktion der beiden Mischungspartner, bei der sich pastöse Massen bilden, die aufgrund ihrer hohen Hygroskopizität zudem leicht zerfließen und daher schlecht zu handhaben sind und sich insbesondere nicht in feste Düngemittelzusammensetzungen einarbeiten lassen.

Magnesiumsulfat-Harnstoff-Verbindungen sind prinzipiell aus der Literatur bekannt. So beschreibt Y. Yee et. al Journal of the American Chemical Society, 1937, 59, 5701 die Herstellung von Magnesiumsulfat-Harnstoff-Komplexen, wobei das Verhältnis von Magnesiumsulfat zu Harnstoff 1 zu 6 bzw. 1 zu 5 beträgt (MgSO₄ • 6 CO(NH₂)₂ • 2 H₂O, MgSO₄ • 5 CO(NH₂)₂ • 2 H₂O). Zur Herstellung dieser Verbindungen werden Bittersalz (MgSO₄ • 7 H₂O) mit Harnstoff in einer alkoholischen Lösung vermengt. Hierbei scheiden sich, je nach stöchiometrischen Verhältnis von Bittersalz zu Harnstoff, die genannten Produkte ab.

C. W. Whittaker et al. Journal of American Society, 1936, 58, 1975 beschreiben Untersuchungen zum Phasendiagramm von Magnesiumsulfat-Harnstoff-Wasser bei 30°C. Als eine der Phasen wird eine Verbindung der Formel MgSO₄ • CO(NH₂)₂ • 3 H₂O postuliert. Zur Herstellung dieser Verbindung werden äquimolare Mengen von Bittersalz (MgSO₄ • 7 H₂O) mit Harnstoff vermengt. Hierbei scheidet sich ein kristallines Produkt ab. Eigene Untersuchungen ergaben, dass es sich hier um ein Gemisch aus der Verbindung der Formel MgSO₄ • CO(NH₂)₂ • 3 H₂O mit Bittersalz (MgSO₄ • 7 H₂O) und ungebundenem Harnstoff und nicht um die Reinsubstanz MgSO₄ • CO(NH₂)₂ • 3 H₂O handelt. Eine Gewinnung der Verbindung MgSO₄ • CO(NH₂)₂ • 3 H₂O aus diesem Gemisch ist nicht möglich, da sich bei Versuchen der Aufreinigung die Verbindung zersetzt.

T. Todorov et. al Acta Crystallographica, Section C: Crystal Structure Communications, 1998, 54(12), 1758 berichten über weitere Magnesiumsulfat-Harnstoff-Komplexe, wobei das Verhältnis von Magnesiumsulfat zu Harnstoff 1 zu 6 (MgSO₄ • 6 CO(NH₂)₂ • 0,5 H₂O) beträgt. Die Herstellung dieses Komplexes erfolgt durch Verdampfung einer wässrigen Lösung bestehend aus entsprechenden molaren Mengen von Bittersalz und Harnstoff.

Z. Fengxing et. al Journal of Inorganic Chemistry, 1997, 13(4), 375, berichtet über Löslichkeits- und Stabilitätsuntersuchungen eines quaternären Systems K₂SO₄ - MgSO₄ - CO(NH₂)₂ - H₂O. Hierbei wurde angeblich eine ternäre Phase der Zusammensetzung MgSO₄ • CO(NH₂)₂ • 2 H₂O identifiziert. Die angegebenen experimentellen Daten des Magnesium-Harnstoff-Komplexes MgSO₄ • CO(NH₂)₂ • 2 H₂O sind jedoch widersprüchlich und nicht nachvollziehbar. Eigene Untersuchungen der Erfinder haben gezeigt, dass z.B. das dort angegebene Diffraktogramm nicht mit dem Diffraktogramm der als MgSO₄ • CO(NH₂)₂ • 2 H₂O identifizierten Substanz übereinstimmt.

W. Xiaolan et al. Acta Physico-Chimica Sinica, 1998, 14(3), 237, berichtet über isotherme Phasendiagramme eines quaternären Systems KCl(K₂SO₄)-MgCl₂ (MgSO₄)-CO(NH₂)₂-H₂O. Hierbei wird in Tabelle 2 erwähnt, dass MgSO₄ • CO(NH₂)₂ • 2 H₂O neben anderen Komplexen als Bestandteil des Bodenkörper identifiziert wurde. Die dort beschriebenen Untersuchungen sind jedoch widersprüchlich, da die dazu angegebenen Lösungs- und Feststoffzusammensetzungen gar kein Sulfat enthalten. Nicht zuletzt aufgrund fehlender spektroskopischer Angaben ist es nicht möglich, die dort beschriebenen Ergebnisse nachzuvollziehen. Eine Isolierung von MgSO₄ • CO(NH₂)₂ • 2 H₂O wird nicht beschrieben.

CH 364 277 beschreibt ein Verfahren zur Herstellung von biuretarmen, magnesiumhaltigen Harnstoffprills. Dabei wird in einer wässrigen Harnstofflösung 6-9 Gew.% Magnesiumsulfat bezogen auf den eingesetzten wasserfreien Harnstoff gelöst und anschließend die Lösung in einem Dünnschichtverdampfer bei Drücken von > 200 Torr und einer Temperatur > 130° C eingedampft. Die erhaltene Schmelze wird dann zu Prills versprüht. Die erhaltenen Magnesiumsulfat enthaltenen Harnstoffprills weisen einen Wassergehalt von 0,6-08 Gew.% auf. Die vergleichsweise Lagerstabilität der Prills ist vermutlich darauf zurückzuführen, dass diese Prills zum einen nahezu wasserfrei sind und zum anderen nur einen sehr geringen Anteil an Magnesiumsulfat aufweisen.

WO 2013/098367 beschreibt die Zusammensetzung und Herstellung von einer Magnesium-Harnstoff-Verbindung der Formel (II), sowie deren Verwendung als Düngemittel oder Düngemittelzusatz. Mischungen verschiedener Magnesium-Harnstoff-Verbindung werden nicht erwähnt.

Der Erfindung liegt daher die Aufgabe zu Grunde, eine Zusammensetzung aus Magnesiumsulfat-Harnstoff bereitzustellen, welche gezielt, einfach, selektiv und ohne störende Nebenprodukte und quantitativ hergestellt werden kann.

Überraschenderweise wurde gefunden, dass mittels geeigneter Verfahren auf schnelle und einfache Weise die Magnesiumsulfat-Harnstoff-Verbindung der Formel (I) sowie Gemische der Verbindung der Formel (I) mit der Verbindung der Formel (II) in sehr guten Ausbeuten als reine, kristalline und lagerstabile Zusammensetzung hergestellt werden können, die die Nachteile des Standes der Technik nicht aufweisen und die insbesondere nur gering hygroskopisch sind und die auch nicht zum Zerfließen neigen. Die so erhaltenen Zusammensetzungen lassen sich daher besonders gut in Düngemittelzusammensetzungen einarbeiten.

Gegenstand der Erfindung sind daher Zusammensetzungen, die wenigstens 80 Gew.-%, insbesondere wenigstens 90 Gew.-%, bezogen auf das Gesamtgewicht der Zusammensetzung, wenigstens einer Magnesiumsulfat-Harnstoff-Verbindung, ausgewählt unter der Verbindung der Formel (I) und Gemischen der Verbindung der Formel (I) mit der Verbindung der Formel (II):

[MgSO₄ • m CO(NH₂)₂ • n H₂O] (I),

[MgSO₄ • x CO(NH₂)₂ • y H₂O] (II),

worin m und x im Bereich von 0,9 bis 1,1, n im Bereich von 1,9 bis < 2,1 und y im Bereich von 2,9 bis 3,1 liegen, enthalten, wobei die Zusammensetzungen, bezogen auf das Gesamtgewicht der Zusammensetzung, weniger als 10 Gew.-%, insbesondere weniger als 5 Gew.-% freies MgSO₄ in Form des Anhydrates oder in Form von Hydraten des Magnesiumsulfats und weniger als 10 Gew.-%, insbesondere weniger als 5 Gew.-% ungebundenen Harnstoff enthalten. Insbesondere weist m in der Formel (I) den Wert 1 auf. Insbesondere weist n in der Formel (I) den Wert 2 auf. Insbesondere weist x in der Formel (II) den Wert 1 auf. Insbesondere weist y in der Formel (II) den Wert 3 auf.

Die erfindungsgemäßen Zusammensetzungen sind feste, in der Regel kristalline, nicht oder nur gering hygroskopische Zusammensetzungen, die außerdem nicht zerfließen. Sie weisen, anders als die aus dem Stand der Technik bekannten Zusammensetzungen der Verbindung der Formel (I) und von Gemischen der Verbindung der Formel (I) mit der Verbindung der Formel (II), nur einen geringen Anteil oder keine nachweisbaren Mengen an Edukten auf. Zudem ist die Verbindung der Formel I alleine, ebenso wie Gemische der Verbindung der Formel (I) mit der Verbindung der Formel (II) in sehr guten Ausbeuten und mit hoher Selektivität zugänglich und in lagerstabiler, gering hygroskopischer Form erhältlich. Außerdem lassen sich diese Zusammensetzungen leicht in Pulverform oder in Form von Granulaten herstellen und daher in einfacher Weise als Düngemittel verwenden oder in konventionelle Düngemittelzusammensetzungen einbringen. Ferner können durch Auflösen der Zusammensetzungen in einem Lösungsmittel, beispielsweise Wasser, Lösungen der Zusammensetzungen hergestellt werden. Weiterhin weisen die erfindungsgemäßen Zusammensetzungen in der Regel nur sehr geringe oder keine nachweisbaren Mengen an Biuret auf. In der Regel beträgt der Anteil an Biuret maximal 1,2 Gew.-%, insbesondere maximal 0,5 Gew.-%, bezogen auf das Gesamtgewicht der Zusammensetzung.

Erfindungsgemäß enthalten die erfindungsgemäßen Zusammensetzungen weniger als 10 Gew.-%, insbesondere weniger als 5 Gew.-%, bezogen auf das Gesamtgewicht der Zusammensetzung, ungebundenen Harnstoff und gleichzeitig weniger als 10 Gew.-%, bezogen auf das Gesamtgewicht der Zusammensetzung, freies MgSO₄ in Form des Anhydrates oder in Form von harnstofffreien Hydraten des Magnesiumsulfats. Vorzugsweise enthält die Zusammensetzung weniger als 5 Gew.-% freies MgSO₄ in Form des Anhydrates oder in Form von harnstofffreien Hydraten des Magnesiumsulfats oder weniger als 5 Gew.-% ungebundenen Harnstoff, jeweils bezogen auf das Gesamtgewicht der Zusammensetzung. Insbesondere enthält die Zusammensetzung weniger als 5 Gew.-%, speziell weniger als 3 Gew.-% freies MgSO₄ in Form des Anhydrates oder in Form von harnstofffreien Hydraten des Magnesiumsulfats und gleichzeitig weniger als 5 Gew.-%, insbesondere weniger als 3 Gew.-% ungebundenen Harnstoff, jeweils bezogen auf das Gesamtgewicht der Zusammensetzung.

Der Anteil an freiem MgSO₄ in Form des Anhydrats oder in Form von Hydraten des Magnesiumsulfats wie Bittersalz, Magnesiumsulfat-Monohydrat oder Magnesiumsulfat-Hexahydrat sowie der Anteil an freiem Harnstoff kann mittels Röntgenpulver-Diffraktometrie durch Vergleich eines Pulverdiffraktogramms mit Referenz-Pulverdiffraktogrammen der Verunreinigungen wie MgSO₄ in Form des Anhydrates, Bittersalz oder auch Magnesiumsulfat-Hexahydrat und Harnstoff ermittelt werden. Derartige Methoden sind dem Fachmann bekannt und können in an sich bekannter Weise, beispielsweise mit Hilfe der Pulverröntgen-Diffraktometrie Software: "EVA" Ver. 12.0.0.0 der Fa. Bruker AXS, Datenbank: Powder Diffraction Files (PDF-2, Release 1999; Data Sets 1-49, plus 70-86) des International Center for Diffraction Data (ICDD) durchgeführt werden.

Aus der Abwesenheit der für die Verunreinigungen charakteristischen Reflexe kann zudem geschlossen werden, dass der Anteil der jeweiligen Verunreinigung gemäß qualitativer Auswertung der RDA Diagramme verschwindend gering ist. Die charakteristischen Reflexe für MgSO₄ in Form des Anhydrats, Bittersalz, Magnesiumsulfat-Hexahydrat sowie Harnstoff können der Literatur oder einschlägigen Datenbanken wie denen des International Centre for Diffraction Data (JCPDS) entnommen werden. Die in den erfindungsgemäßen Zusammensetzungen enthaltene Verbindung der Formel (I) weist in einem bei 25 °C aufgenommenen Röntgenpulverdiffraktogramm (Cu-K_{α}-Strahlung: λ = 1.5413 Å) wenigstens 3 und insbesondere wenigstens 5 und speziell wenigstens 7 oder alle d-Werte der folgenden Tabelle 1 auf, wobei es vorzugsweise wenigstens 3, insbesondere wenigsten 5 und speziell wenigstens 7 derjenigen Reflexe aufweist, deren relative Intensität größer 8 %, bezogen auf die Intensität des stärksten Peaks (100 % rel. Intensität) ist. In Tabelle 1 sind die charakteristischen Reflexe der Verbindung (I) als Netzbenennungsabstände d (in Ångström) angegeben, die sich aus der Bragg'schen Beziehung 2θ-Werten berechnen lassen.

**Tabelle 1. Verbindung Formel (I)**

| **d-Wert (Å)** | **rel. Intensität (%)** |
|---|---|
| 6,937 | 100 |
| 3,349 | 45 |
| 3,157 | 43 |
| 3,257 | 28 |
| 3,364 | 25 |
| 3,469 | 23 |
| 4,940 | 19 |
| 4,917 | 17 |
| 2,605 | 12 |
| 3,287 | 11 |
| 2,621 | 8 |
| 2,100 | 7 |
| 2,314 | 6 |
| 2,473 | 6 |
| 4,180 | 6 |
| 2,900 | 5 |
| 2,567 | 5 |
| 9,637 | 5 |
| 2,434 | 4 |
| 2,031 | 4 |
| 1,955 | 4 |
| 4,611 | 3 |
| 2,540 | 3 |
| 1,736 | 3 |

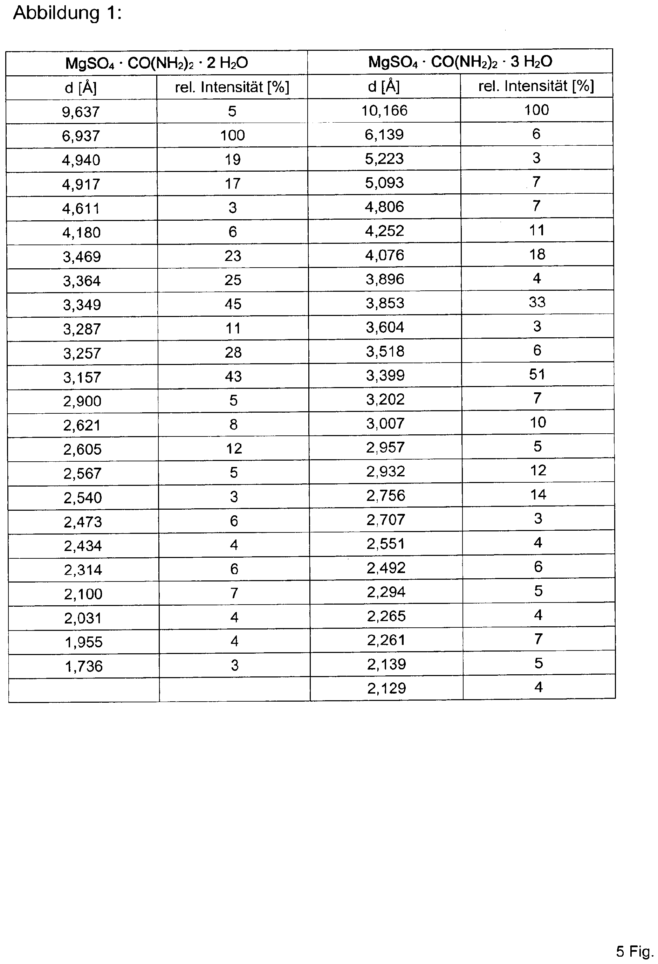

Die in den erfindungsgemäßen Zusammensetzungen enthaltenen Verbindungen können in einem bei 25 °C (Cu-K_{α}-Strahlung: λ = 1.5413 Å) aufgenommenen Röntgenpulverdiffraktogramm der Zusammensetzung anhand der in der folgenden Tabelle 2 angegebene Reflexe identifiziert werden, wobei zur Identifikation typischerweise wenigstens 3 und insbesondere wenigstens 5 der angegebenen d-Werte mit einer relativen Intensität größer 10 % herangezogen werden. Eine vollständige Auflistung der d-Werte ist in Abbildung 1 dargestellt.

**Tabelle 2**

| **Verunreinigung** | **d-Wert (Å)** | **rel. Intensität (%)** |
|---|---|---|
| Harnstoff | 3,9916 | 100 |
| | 3,6138 | 24 |
| | 3,0435 | 25 |
| MgSO₄ · 7 H₂O | 4,2160 | 100 |
| | 4,2000 | 75 |
| | 5,3400 | 30 |
| | 5,9800 | 30 |
| MgSO₄ · H₂O | 3,4050 | 100 |
| | 4,8150 | 75 |
| | 3,3510 | 70 |
| | 3,3130 | 70 |
| MgSO₄ | 3,5300 | 100 |
| | 3,6100 | 70 |
| | 4,1500 | 30 |

Die Stöchiometrie der Verbindung kann durch Elementaranalyse unter Berücksichtigung der vorhandenen Verunreinigungen bestimmt werden.

Eine erste bevorzugte Ausführungsform der Erfindung betrifft Zusammensetzungen mit einem hohen Anteil an der Verbindung der Formel (I), insbesondere Zusammensetzungen, worin der Anteil der Verbindung der Formel (I), bezogen auf die Gesamtmenge an Verbindungen der Formeln (I) und (II), wenigstens 90 Gew.-% insbesondere wenigstens 95 Gew.-% beträgt und speziell solche Zusammensetzungen, die keine nachweisbaren Mengen an der Verbindung der Formel (II) enthalten.

Eine Ausführungsform betrifft Zusammensetzungen, welche ein Gemisch aus der Verbindung der Formel (I) und der Verbindung der Formel (II) enthalten, insbesondere Zusammensetzungen, worin der Anteil der Verbindung der Formel (I), bezogen auf die Gesamtmenge an Verbindungen der Formeln (I) und (II), im Bereich von 20 bis 90 Gew.-%, insbesondere im Bereich von 30 bis 85 Gew.-% liegt.

Es wurde weiterhin ein Verfahren zur Herstellung der erfindungsgemäßen Zusammensetzungen der Magnesiumsulfat-Harnstoff-Verbindung der Formel (I) und Gemischen der Verbindung der Formel (I) mit der Verbindung der Formel (II) gefunden, das dadurch gekennzeichnet ist, dass man wasserfreies Magnesiumsulfat mit Harnstoff und Wasser umsetzt. Dieses Verfahren wird im Folgenden auch als Verfahren 1 bezeichnet. Das Verfahren 1 ermöglicht die Herstellung vor allem von erfindungsgemäßen Zusammensetzungen mit einem hohen Anteil an der Verbindung der Formel (I), insbesondere die Herstellung von Zusammensetzungen, worin der Anteil der Verbindung der Formel (I), bezogen auf die Gesamtmenge an Verbindungen der Formeln (I) und (II), wenigstens 90 Gew.-% insbesondere wenigstens 95 Gew.-% beträgt und speziell von solchen Zusammensetzungen, die keine nachweisbaren Mengen an der Verbindung der Formel (II) enthalten. Das Verfahren 1 ermöglicht durch Variation der Reaktionsbedingungen, wie unten erläutert, auch die Herstellung von Zusammensetzungen, welche ein Gemisch aus der Verbindung der Formel (I) und der Verbindung der Formel (II) enthalten.

Vorzugsweise wird wasserfreies Magnesiumsulfat in Form eines Pulvers eingesetzt, worin wenigstens 90 Gew.-%, insbesondere wenigstens 95 Gew.-% der Partikel einen Teilchendurchmesser kleiner 200 µm, insbesondere maximal 100 µm aufweisen. Derartige Pulver können durch Aufmahlung von handelsüblichem, wasserfreiem Magnesiumsulfat hergestellt werden. Vorzugsweise wird Harnstoff in Form eines Granulats eingesetzt, worin wenigstens 90 Gew.-%, insbesondere wenigstens 95 Gew.-% der Partikel einen Teilchendurchmesser kleiner 1 mm aufweisen. Derartige Pulver bzw. Granulate können durch Aufmahlung von handelsüblichem, geprilltem Harnstoff hergestellt werden. Der Teilchendurchmesser der Pulverteilchen des Magnesiumsulfats kann ebenso wie der Teilchendurchmesser des Harnstoffpulvers bzw. -granulats durch Siebanalyse bestimmt werden. Der Einsatz von handelsüblichem, geprilltem Harnstoff führt in der Regel zu Zusammensetzungen mit hohen Anteilen an Verbindung der Formel (II).

Erfindungsgemäß setzt man im Verfahren 1 wasserfreies Magnesiumsulfat, Harnstoff und Wasser in der für die Verbindung in einem Molverhältnis von Magnesiumsulfat zu Harnstoff im Bereich von 1 : 0,9 bis 1 : 1,1, insbesondere 1 : 0,95 bis 1 : 1,05 und speziell 1 : 0,98 bis 1 : 1,02 und einem Verhältnis von Magnesiumsulfat zu Wasser im Bereich von 1 : 2,0 bis 1: 4,0, insbesondere 1 : 2,95 bis 1 : 3,5 und speziell 1 : 2,98 bis 1 : 3,02.

Insbesondere wird man zur Umsetzung gemäß Verfahren 1 wasserfreies Magnesiumsulfat mit Harnstoff im obengenannten Molverhältnis vermischen und hierzu Wasser im obengenannten Molverhältnis geben. Eine mögliche Ausgestaltung des Verfahrens 1 wird in Beispiel 1 beschrieben.

Die Umsetzung gemäß Verfahren 1 erfolgt bei Temperaturen oberhalb 75°C. Insbesondere wird man die Umsetzung bei Temperaturen im Bereich von 80 bis 95°C durchführen. Vorzugsweise wird man dabei eine Temperatur von 95 °C nicht überschreiten. Temperaturen von oberhalb 95°C können zur unerwünschten Bildung von Biuret führen. Vorzugsweise wird man während der Umsetzung die in der Regel flüssige oder plastische Reaktionsmischung durchmischen, beispielsweise mittels geeigneter Rühr- oder Knetvorrichtungen oder mittels Extruder. Typischerweise wird die vorerst flüssige oder plastische Reaktionsmischung mit fortschreitender Reaktionsdauer fest.

Nach der Umsetzung der Edukte lässt man diese in der Regel auf Umgebungstemperatur abkühlen. Es hat sich als vorteilhaft erwiesen, wenn man die erhaltene Zusammensetzung unmittelbar im Anschluss an die Umsetzung der Edukte bzw. im Anschluss an das Abkühlen für eine gewisse Zeit bei einer Temperatur im Bereich von 15 bis 40°C, insbesondere 20 bis 30°C reifen lässt, bevor sie weiterbehandelt wird. Die Zeitdauer der Reifung wird in der Regel im Bereich von 15 min bis 20 h, vorzugsweise im Bereich von 2 bis 12 Stunden, insbesondere im Bereich von 3 bis 8 Stunden liegen.

Weiterhin hat sich als besonders vorteilhaft erwiesen, wenn man die Reaktion in einer Mischvorrichtung mit wenigstens einem rotierenden Mischwerkzeug, beispielsweise eines Kneters oder Intensivmischers (z.B. eines Eirich-Mischers), durchführt. Damit kann bereits während oder direkt im Anschluss an die Umsetzung durch eine Drehzahlerhöhung der Mischwerkzeuge eine Zerkleinerung und/oder Granulation des Reaktionsgemisches und eine Einstellung der gewünschten Korngrößen bzw. Teilchendurchmesser vorgenommen werden. Die Drehzahl des Mischers wird hierbei innerhalb von wenigen Minuten nach Beginn des Abbindevorganges (erkennbar an dem Auftreten eine pastösen Masse) erhöht. Nach Ende der Umsetzung (ca. 3 bis 30 min) und Entleerung des Mischers, reift das Produkt - z.B. wie oben beschrieben - nach.

Gemäß einer bevorzugten Ausführungsform des Verfahrens 1 wird die nach dem Reifungsprozess erhaltene feste Zusammensetzung zerkleinert. Für die Zerkleinerung der Zusammensetzung können übliche Vorrichtungen zum Zerkleinern von Feststoffen eingesetzt werden, vorzugsweise Schlag- oder Prallmühlen, unter anderem auch Backenbrecher, Rundbrecher, Walzenbrecher, Hammerbrecher, Daumenbrecher, Schneckenbrecher, Wälzmühlen, Schlag- und Schleudermühlen. Üblicherweise wird man die Zerkleinerung so lange durchführen, bis mindestens 90 Gew.-% der Partikel einen Durchmesser kleiner 5 mm aufweisen. Vorzugsweise wird man das Zerkleinern so durchführen, dass wenigstens 90 Gew.-% der Partikel einen Teilchendurchmesser im Bereich von 0,1 bis 5 mm aufweisen. Der Teilchendurchmesser kann durch Siebanalyse bestimmt werden.

Ein weiterer Vorteil des Verfahrens 1 besteht in der Möglichkeit, bei Einsatz einer geeigneten Mischvorrichtung, beispielsweise eines Kneters oder Intensivmischers, z.B. eines Eirich-Mischers, bereits während der Umsetzung in einer Mischvorrichtung mit wenigstens einem rotierenden Mischwerkzeug, durch Drehzahlerhöhung der Mischwerkzeuge eine Zerkleinerung und/oder Granulierung des Reaktionsgemisches und damit eine Einstellung der oben genannten Korngrößen bzw. Teilchendurchmesser durchzuführen. Auch die Reifung der Verbindung der Formel (I) geschieht dabei vorteilhafterweise relativ schnell (innerhalb von Stunden).

Das erfindungsgemäße Verfahren 1 und das weiter unten beschriebene Verfahren 2 ermöglichen es, die Verbindung der Formel (I) und Gemische der Verbindung der Formel (I) mit der Verbindung der Formel (II) in hoher Ausbeute, in der Regel praktisch zu wenigstens 90 %, insbesondere wenigstens 95 bis 100 %, bezogen auf Magnesiumsulfat und Harnstoff herzustellen. Der Anteil an freiem Magnesiumsulfat, sei es in Form des Anhydrats oder eines harnstofffreien Hydrates in der erhaltenen Zusammensetzung beträgt weniger als 10 Gew.-%, häufig weniger als 5 Gew.-%, insbesondere weniger als 3 Gew.-%. Des Weiteren ermöglicht das erfindungsgemäße Verfahren, Zusammensetzungen der Verbindung der Formel (I), die weniger als 10 Gew.-%, häufig weniger als 5 Gew.-% und insbesondere weniger als 3 Gew.-% an ungebundenem Harnstoff enthalten, herzustellen. Die so hergestellten Zusammensetzungen enthalten, wenn überhaupt, nur einen sehr geringen Anteil an Biuret von in der Regel maximal 1,2 Gew.-%, insbesondere maximal 0,5 Gew.-%, bezogen auf das Gesamtgewicht der Zusammensetzung.

Anders als im Verfahren des Standes der Technik erfordern die erfindungsgemäßen Verfahren 1 und 2 keine zusätzlichen Lösungsmittel bzw. Filtrations- und Trocknungsschritte und sind daher besonders einfach und mit hoher Energie-Effizienz durchführbar.

Neben dem zuvor beschriebenen Verfahren 1 wurde weiterhin ein Verfahren 2 zur Herstellung der erfindungsgemäßen Zusammensetzungen von Magnesiumsulfat-Harnstoff-Verbindungen der Formel (I) und Gemischen der Verbindung der Formel (I) mit der Verbindung der Formel (II) gefunden, das dadurch gekennzeichnet ist, dass man eine Zusammensetzung, die wenigstens 80 Gew.-%, insbesondere wenigstens 90 Gew.-%, bezogen auf das Gesamtgewicht der Zusammensetzung, der Magnesiumsulfat-Harnstoff-Verbindung der Formel (II) und weniger als 10 Gew.-%, insbesondere weniger als 5 Gew.-% und speziell weniger als 3 Gew.-% freies MgSO₄ in Form des Anhydrates oder in Form von Hydraten des Magnesiumsulfats und weniger als 10 Gew.-%, insbesondere weniger als 5 Gew.-% und speziell weniger als 3 Gew.-% ungebundenen Harnstoff enthält, bei Temperaturen im Bereich von 60 bis 95°C, insbesondere bei Temperaturen im Bereich von 65 bis 85 °C trocknet. Dieses Verfahren wird im Folgenden als Verfahren 2 bezeichnet. Eine mögliche Ausgestaltung des Verfahrens 2 wird in Beispiel 2 beschrieben.

Das Verfahren 2 ermöglicht die Herstellung sowohl von erfindungsgemäßen Zusammensetzungen mit einem hohen Anteil an der Verbindung der Formel (I), insbesondere die Herstellung von Zusammensetzungen, worin der Anteil der Verbindung der Formel (I), bezogen auf die Gesamtmenge an Verbindungen der Formeln (I) und (II), wenigstens 90 Gew.-% insbesondere wenigstens 95 Gew.-% beträgt und speziell von solchen Zusammensetzungen, die keine nachweisbaren Mengen an der Verbindung der Formel (II) enthalten, als auch von Zusammensetzungen, welche ein Gemisch aus der Verbindung der Formel (I) und der Verbindung der Formel (II) enthalten. Der Anteil an Verbindung (I) kann in einfacher Weise durch die Dauer der Umsetzung gesteuert werden.

Vorzugsweise wird zur Herstellung der erfindungsgemäßen Zusammensetzungen nach dem Verfahren 2 eine Magnesiumsulfat-Harnstoff-Verbindung der Formel (II)

[MgSO₄ • x CO(NH₂)₂ • y H₂O] (II),

worin x im Bereich von 0,9 bis 1,1 und y im Bereich von 2,9 bis 3,1 liegt, in Form einer Zusammensetzung eingesetzt, die, bezogen auf ihr Gesamtgewicht, weniger als 10 Gew.-%, insbesondere weniger als 5 Gew.-% und speziell weniger als 3 Gew.-% freies MgSO₄ in Form des Anhydrates oder in Form von Hydraten des Magnesiumsulfats und/oder weniger als 10 Gew.-%, insbesondere weniger als 5 Gew.-% und speziell weniger als 3 Gew.-% ungebundenen Harnstoff. Insbesondere weist x in der Formel (II) den Wert 1 auf. Insbesondere weist y in der Formel (II) den Wert 3 auf.

Die in den Zusammensetzungen enthaltene Verbindung der Formel (II) weist in einem bei 25 °C aufgenommenen Röntgenpulverdiffraktogramm (Cu-K_{α}-Strahlung: λ = 1.5413 Å) wenigstens 3 und insbesondere wenigstens 5 und speziell wenigstens 7 oder alle d-Werte der folgenden Tabelle 3 auf, wobei es vorzugsweise wenigstens 3, insbesondere wenigsten 5 und speziell wenigstens 7 derjenigen Reflexe aufweist, deren relative Intensität größer 8 %, bezogen auf die Intensität des stärksten Peaks (100 % rel. Intensität) ist. In Tabelle 3 sind die charakteristischen Reflexe der Verbindung (II) als Netzbenennungsabstände d (in Angström) angegeben, die sich aus der Bragg'schen Beziehung 2Θ-Werten berechnen lassen.

**Tabelle 3**

| **d-Wert (Å)** | **rel. Intensität (%)** |
|---|---|
| 10,1656 | 100 |
| 3,3994 | 63 |
| 3,8527 | 39 |
| 4,0752 | 21 |
| 2,9318 | 15 |
| 2,7558 | 15 |
| 4,2520 | 13 |
| 3,0064 | 13 |
| 4,8050 | 9 |
| 3,2015 | 9 |
| 5,0928 | 8 |
| 2,2609 | 8 |
| 2,4920 | 8 |
| 6,1384 | 7 |
| 3,5177 | 7 |
| 2,5505 | 7 |
| 2,2649 | 6 |
| 2,9572 | 6 |
| 2,2944 | 6 |
| 2,1393 | 6 |
| 2,1293 | 6 |
| 2,4767 | 5 |

Vorzugsweise wird im Verfahren 2 eine Zusammensetzung der Verbindung der Formel (II) in Form eines Pulvers oder Granulats eingesetzt, worin wenigstens 90 Gew.-% der Partikel einen Teilchendurchmesser kleiner 5 mm aufweisen. Der Teilchendurchmesser kann durch Siebanalyse bestimmt werden.

Die Herstellung von Zusammensetzungen der Verbindung der Formel (II) ist in WO 2013/ 098 367 beschrieben.

Zusammensetzungen der Verbindung der Formel (II) können durch die Umsetzung von wasserfreiem Magnesiumsulfat, Harnstoff und Wasser in der für die Verbindung erforderlichen Stöchiometrie hergestellt werden. Die Umsetzung erfolgt in der Regel bei Temperaturen unterhalb 70°C, vorzugsweise bei Temperaturen von maximal 60°C, insbesondere bei Temperaturen im Bereich von 30 bis 60 °C. Zur Herstellung von Zusammensetzungen der Verbindungen der Formel (II) werden in der Regel Magnesiumsulfat, Harnstoff und Wasser in der für die Verbindung erforderlichen Stöchiometrie miteinander umgesetzt, d. h. in einem Molverhältnis von Magnesiumsulfat zu Harnstoff im Bereich von 1 : 0,9 bis 1 : 1,1, insbesondere 1 : 0,95 bis 1 : 1,05 und speziell 1 : 0,98 bis 1 : 1,02 und einem Verhältnis von Magnesiumsulfat zu Wasser im Bereich von 1 : 2,9 bis 1: 3,1, insbesondere 1 : 2,95 bis 1 : 3,05 und speziell 1 : 2,98 bis 1 : 3,02.

Diese Umsetzung von wasserfreiem Magnesiumsulfat, Harnstoff und Wasser zur Herstellung von Zusammensetzungen der Verbindung der Formel (II) ist exotherm. Hierbei hat es sich als vorteilhaft erwiesen, die Umsetzung der Reaktionspartner wasserfreies Magnesiumsulfat, Harnstoff und Wasser unter Ausnutzung der Reaktionswärme durchzuführen. Vorzugsweise wird man dabei eine Temperatur von 70 °C, insbesondere 60°C nicht überschreiten. Gegebenenfalls wird man daher das Reaktionsgemisch kühlen. Insbesondere erfolgt die Umsetzung bei Temperaturen im Bereich von 30 bis 60 °C. Gegebenenfalls wird man Maßnahmen treffen, um Wasser, welches während der Umsetzung verdampft, in die Reaktion zurückzuführen. Vorzugsweise wird man während der Umsetzung die in der Regel flüssige oder plastische Reaktionsmischung durchmischen, beispielsweise mittels geeigneter Rühr- oder Knetvorrichtungen oder mittels Extruder. Nach Beendigung der Reaktion lässt man die entstandene Zusammensetzung in der Regel auf Umgebungstemperatur abkühlen. Hierbei erhält man typischerweise eine feste Masse, die in an sich bekannter Weise zerkleinert werden kann.

Eine weitere Möglichkeit zur Herstellung von Zusammensetzungen der Verbindung der Formel (II) besteht darin, dass man zunächst eine Mischung Bittersalz (MgSO₄ • 7 H₂O) mit Harnstoff im Molverhältnis Bittersalz zu Harnstoff im Bereich von 1 : 0,9 bis 1 : 1,1, insbesondere 1 : 0,95 bis 1 : 1,05 und speziell 1 : 0,98 bis 1 : 1,02 umsetzt und zu der erhaltenen Mischung im Verlauf der Umsetzung weiteren Harnstoff zugibt. Vorzugsweise gibt man im Verlauf der Umsetzung zur Herstellung der Verbindungen der Formel (II) weiteren Harnstoff in einer Menge von 10 bis 30 mol-%, bezogen auf die zunächst eingesetzte Harnstoffmenge, zu dem Gemisch aus Bittersalz (MgSO₄ • 7 H₂O) und Harnstoff. Die Zugabe kann in einer Portion oder in mehreren Portionen oder auch kontinuierlich erfolgen. Die Zugabe des weiteren Harnstoffs erfolgt in der Regel zu einem Zeitpunkt, an dem Bittersalz (MgSO₄ • 7 H₂O) und Harnstoff bereits miteinander vermischt wurden. Vorzugsweise erfolgt die Umsetzung bei Temperaturen im Bereich von 20 bis 70 °C und insbesondere im Bereich von 40 bis 60 °C. Da die Umsetzung kaum Wärmetönung aufweist, wird man gegebenenfalls das Gemisch aus Bittersalz und Harnstoff auf die gewünschte Temperatur erwärmen. Vorzugsweise wird man während der Umsetzung die in der Regel flüssige oder plastische Reaktionsmischung durchmischen, beispielsweise mittels geeigneter Rühr- oder Knetvorrichtungen. Auf diese Weise wird ein Reaktionsgemisch in Form einer wässrigen Suspension erhalten. Hieraus können die Verbindung der Formel (II) bzw. die sie enthaltende Zusammensetzung durch übliche Methoden der Trennung von festen und flüssigen Phasen isoliert werden, beispielsweise durch Filtration oder Zentrifugation. In der Regel wird man einen Trocknungsschritt anschließen. Vorzugsweise erfolgt die Trocknung bei Temperaturen im Bereich von 30 bis 70 °C und insbesondere im Bereich von 30 bis 50 °C. Die so hergestellten Verbindungen der Formel (II) fallen typischerweise in feinteiliger Form an.

Eine weitere Möglichkeit zur Herstellung von Verbindungen der Formel (II) besteht darin, dass man zunächst eine Mischung von wasserfreiem Magnesiumsulfat, Harnstoff und Bittersalz (MgSO₄ • 7 H₂O) umsetzt. Bei dieser Vorgehensweise wird vorzugsweise wasserfreies Magnesiumsulfat, Harnstoff und Bittersalz in der für die Verbindung erforderlichen Stöchiometrie ein, d. h. in einem Molverhältnis wasserfreies Magnesiumsulfat : Harnstoff im Bereich von 1 : 1,65 bis 1 : 1,85 und das Molverhältnis wasserfreies Magnesiumsulfat: Bittersalz im Bereich von 1 : 0,65 bis 1 : 0,85, wobei das Molverhältnis von Harnstoff zur Gesamtmenge an wasserfreiem Magnesiumsulfat und Bittersalz im Bereich von 0,9 : 1 bis 1,1 : 1 liegt. Vorzugsweise wird zunächst die Mischung der obengenannten Edukte auf 30 bis 60 °C, insbesondere auf 40 bis 55 °C, erwärmt. Vorzugsweise wird man während der Umsetzung die in der Regel flüssige oder plastische Reaktionsmischung durchmischen, beispielsweise mittels geeigneter Rühr- oder Knetvorrichtungen oder mittels Extruder. Die entstehende pastöse Masse der entstandenen Zusammensetzung erstarrt nach Abkühlung auf Umgebungstemperatur. Hierbei erhält man typischerweise eine feste Masse, die in an sich bekannter Weise zerkleinert werden kann.

Vorzugsweise wird im Verfahren 2 die Zusammensetzung der Magnesiumsulfat-Harnstoff-Verbindung der Formel (II), erhältlich nach einen der oben beschriebenen Verfahren, 10 bis 30 Stunden, insbesondere 15 bis 28 Stunden bei Temperaturen im Bereich von 65 bis 95°C, insbesondere im Bereich von 65 bis 85 °C getrocknet. Temperaturen von oberhalb 95°C wird man in der Regel nicht anwenden, da sie zur unerwünschten Bildung von Biuret führen können. Es ist möglich, während der Trocknung Proben zu entnehmen, um den Endpunkt, der vollständigen Umsetzung durch gravimetrische oder röntgenanalytische Untersuchungen festzustellen.

Es ist möglich die Zusammensetzung der Verbindung der Formel (II) während der Erwärmung zu durchmischen, beispielsweise mittels geeigneter Rühr- oder Knetvorrichtungen.

Die nach Verfahren 2 erhältliche erfindungsgemäße Zusammensetzung der Verbindung der Formel (I) und Gemische der Verbindung der Formel (I) mit der Verbindung der Formel (II) fällt typischerweise in feinteiliger Form an, worin weniger als 90 Gew.-% der Partikel einen Durchmesser oberhalb 5 mm aufweisen. Vorzugsweise weisen wenigstens 90 Gew.-% der Partikel einen Teilchendurchmesser kleiner 2 mm, insbesondere bevorzugt kleiner 1 mm, auf. Der Teilchendurchmesser kann durch Siebanalyse bestimmt werden.

Das erfindungsgemäße Verfahren 2 ermöglicht es ebenfalls, die Verbindung der Formel (I) alleine oder aber Gemische der Verbindung der Formel (I) mit der Verbindung der Formel (II) in hoher Ausbeute, in der Regel wenigstens 70 %, insbesondere wenigstens 80 %, speziell wenigstens 90 %, bezogen auf Magnesiumsulfat, herzustellen. Der Anteil an freiem Magnesiumsulfat, sei es in Form des Anhydrats oder eines harnstofffreien Hydrates, in der so erhaltenen Zusammensetzung beträgt weniger als 10 Gew.-% und insbesondere weniger als 5 Gew.-% und speziell weniger als 3 Gew.-%, bezogen auf das Gesamtgewicht der Zusammensetzung. Der Anteil an freiem Harnstoff in der so erhaltenen Zusammensetzung beträgt weniger als 10 Gew.-% und insbesondere weniger als 5 Gew.-% und speziell weniger als 3 Gew.-%, bezogen auf das Gesamtgewicht der Zusammensetzung. Die so hergestellten Zusammensetzungen enthalten, wenn überhaupt, nur einen sehr geringen Anteil an Biuret von in der Regel maximal 1,2 Gew.-%, insbesondere maximal 0,5 Gew.-%, bezogen auf das Gesamtgewicht der Zusammensetzung.

Die Erfindung betrifft weiterhin die Verwendung der erfindungsgemäßen Zusammensetzungen als Düngemittel oder als Düngemitteladditiv.

Die erfindungsgemäßen Zusammensetzungen sind als solche als Düngemittel geeignet und stellen für sich eine Stickstoff-Magnesium-Schwefel Düngemittelzusammensetzung dar. Sie eignet sich jedoch insbesondere auch als Stickstofflieferant für konventionelle Magnesium-Schwefel-Düngemittelzusammensetzungen. Durch Additivierung konventioneller Magnesium-Schwefel-Düngemittelzusammensetzungen können somit in einfacher Weise Stickstoff-Magnesium-Schwefel-Düngemittelzusammensetzungen bereitgestellt werden.

Derartige Stickstoff-Magnesium-Schwefel-Düngemittelzusammensetzungen enthalten die Verbindung der Formel (I) oder Gemische der Verbindung der Formel (I) mit Verbindung der Formel (II), wobei der Gesamtgehalt von Verbindung (I) und Verbindung (II) typischerweise in einer Menge von 1 bis 100 Gew.-%.

Neben der Verbindung der Formel (I) oder den Gemischen der Verbindung der Formel (I) mit Verbindung der Formel (II) und gegebenenfalls Magnesiumsulfat, vorzugsweise in Form von Bittersalz, können die Stickstoff-Magnesium-Schwefel-DüngemittelZusammensetzungen weitere Makronährstoffe wie Phosphor, vorzugsweise in Form von Phosphaten, und/oder Kalium, sowie gegebenenfalls Mikronährstoffe wie Mangan, Zink, Kupfer, Eisen, Kobalt, Molybdän und/oder Bor enthalten. Mangan und Zink werden dabei vorzugsweise in Form ihrer Sulfate eingesetzt. Kupfer, Kobalt und Eisen werden vorzugsweise in Form von Chelaten, z. B. mit EDTA, eingesetzt. Bor wird vorzugsweise als Natriumborat oder Borsäure eingesetzt. Molybdän wird vorzugsweise als Natrium- oder Ammoniummolybdat oder als Mischung davon eingesetzt.

Vorzugsweise werden die erfindungsgemäßen Zusammensetzungen als Additiv in Bittersalz enthaltenden Düngemitteln verwendet. Derartige Düngemittel bzw. Düngemittelzusammensetzungen sind neu und ebenfalls Gegenstand der Erfindung. Insbesondere enthält die erfindungsgemäße Düngemittelzusammensetzung neben Bittersalz 1 bis 90 Gew.-%, insbesondere 5 bis 50 Gew.-% und speziell 10 bis 30 Gew.-%, bezogen auf das Gesamtgewicht der Düngemittelzusammensetzung, der Verbindung der Formel (I) und Verbindung der Formel (II) sowie gegebenenfalls ein oder mehrere der vorgenannten Mikronährstoffe. Der Anteil der Mikronährstoffe, gerechnet als Element, wird in der Regel nicht mehr als 30 Gew.-%, bezogen auf das Gesamtgewicht der Düngemittelzusammensetzung, betragen und liegt, sofern erwünscht, häufig im Bereich von 0,1 bis 10 Gew.-%.
Die erfindungsgemäßen Zusammensetzungen der Verbindung der Formel (I) bzw. der Gemische der Verbindung der Formel (I) mit Verbindung der Formel (II) können auch gemeinsam mit sogenannten Nitrifikationsinhibitoren und/oder Ureaseinhibitoren verwendet werden. Dabei können die erfindungsgemäßen Zusammensetzungen der Verbindung der Formel (I), bzw. der Gemische der Verbindung der Formel (I) mit Verbindung der Formel (II), im Gemisch mit Nitrifikationsinhibitoren oder im Gemisch mit Ureaseinhibitoren oder im Gemisch Nitrifikationsinhibitoren und Ureaseinhibitoren verwendet werden.

Düngemittelzusammensetzungen, die neben wenigstens einer Verbindung der Formel (I) oder einem Gemisch der Verbindung der Formel (I) mit Verbindung der Formel (II) in Form der erfindungsgemäßen Zusammensetzung und wenigstens einen weiteren Bestandteil enthalten, der unter Ureaseinhibitoren und Nitrifikationsinhibitoren ausgewählt ist, sind ebenfalls Gegenstand der vorliegenden Erfindung.

Geeignete Ureaseinhibitoren sind dem Fachmann bekannt, beispielsweise aus Kiss et al. (Kiss, S., Simihäian, M. 2002, Improving Efficiency of Urea Fertilizers by Inhibition of Soil Urease Activity, ISBN 1-4020-0493-1, Kluwer Academic Publishers, Dordrecht, The Netherland). Geeignete Ureaseinhibitioren sind vor allem N-Alkylphosphorsäuretriamide und N-Alkylthiophosphorsäuretriamide und deren Gemischen, wie sie z. B. aus WO 2009/079994 und der dort zitierten Literatur bekannt sind. Bevorzugt sind N-n-Butylthiophosphorsäuretriamid (NBPT), N-n-Propylthiophosphorsäuretriamid (NPPT) und deren Gemische.

Geeignete Nitrifikationsinhibitoren sind neben Dicyandiamid vor allem Pyrazole und deren Säureadditionssalzen, insbesondere deren Phosphorsäureadditionssalze, sowie 1-Carboxyalkylpyrazole und deren Gemische. Hierbei können die Pyrazole und 1-Carboxyalkylpyrazole an den Kohlenstoffatomen durch ein oder mehrere, z. B. ein oder zwei Substituenten aus der Gruppe C₁-C₄-Alkyl, insbesondere Methyl, Nitro und Halogen, insbesondere Chlor, substituiert sein. Derartige Verbindungen und ihre Verwendung als Nitrifikationsinhibitoren sind beispielsweise aus der US 3635690, der US 4969946, der EP 0808298 und der EP 1120388. Bevorzugte Nitrifikationsinhibitoren sind 3-Methyl-pyrazolverbindungen wie 4-Chlor-3-methylpyrazol und dessen Säureadditionssalze, N-Hydroxymethyl-4-chlor-3-methylpyrazol und dessen Säureadditionssalze, sowie 3,4-Dimethyl-pyrazol (DMP)-Verbindungen wie 2-(3,4-Dimethylpyrazol-1-yl)-bernsteinsäure, N-Hydroxy-methyl-3,4-dimethylpyrazol und dessen Säureadditionssalze sowie vor allem 3,4-Dimethylpyrazol und die Säureadditionssalze des 3,4-Dimethylpyrazols, speziell seine Phosphorsäureadditionssalze (DMPP).

Derartige Düngemittelzusammensetzungen enthalten die Verbindung der Formel (I) oder ein Gemisch der Verbindung der Formel (I) mit Verbindung der Formel (II) und wenigstens einen weiteren Bestandteil aus der Gruppe der Nitrifikationsinhibitoren und Ureaseinhibitoren in der Regel in einer Menge von 0,001 bis 5 Gew.-%, insbesondere in einer Menge von 0,002 bis 3 Gew.-%, bezogen auf das Gesamtgewicht der Düngemittelzusammensetzung.

Sofern derartige Düngemittelzusammensetzungen wenigstens einen Ureaseinhibitor enthalten, beträgt die Konzentration an Ureaseinhibitor in der Regel 0,001 bis 3 Gew.-%, insbesondere 0,002 bis 2 Gew.-%, bezogen den Harnstoff in der Düngemittelzusammensetzung.

Sofern derartige Düngemittelzusammensetzungen wenigstens einen Nitrifikationsinhibitor enthalten, beträgt die Konzentration an Nitrifikationsinhibitor in der Regel 0,01 bis 3 Gew.-%, insbesondere 0,02 bis 2 Gew.-%, bezogen auf das Gesamtgewicht der Düngemittelzusammensetzung, im Falle von Säureadditionssalzen von Pyrazolverbindungen, gerechnet als Salz.

Sofern derartige Düngemittelzusammensetzungen wenigstens einen Ureaseinhibitor und wenigstens einen Nitrifikationsinhibitor enthalten, beträgt die Gesamtkonzentration an Nitrifikationsinhibitor + Ureaseinhibitor in der Regel 0,011 bis 5 Gew.-%, insbesondere 0,022 bis 3 Gew.-%, bezogen auf das Gesamtgewicht der Düngemittelzusammensetzung. Typischerweise beträgt dann das Gewichtsverhältnis von dem wenigstens einen Nitrifikationsinhibitor zu dem wenigstens einen Ureaseinhibitor in der Regel 1 : 10 bis 10 : 1 und vorzugsweise 1 : 5 bis 5 : 1.

Düngemittelzusammensetzungen, die neben der Verbindung der Formel (I) oder einem Gemisch der Verbindung der Formel (I) mit Verbindung der Formel (II) in Form der erfindungsgemäßen Zusammensetzung wenigstens einen weiteren Bestandteil enthalten, der unter Ureaseinhibitoren und Nitrifikationsinhibitoren ausgewählt ist, können außerdem gegebenenfalls Magnesiumsulfat, vorzugsweise in Form von Bittersalz, und/oder weitere Makronährstoffe wie Phosphor, vorzugsweise in Form von Phosphaten, und/oder Kalium, sowie gegebenenfalls Mikronährstoffe wie Mangan, Zink, Kupfer, Eisen, Kobalt, Molybdän und/oder Bor enthalten. Mangan und Zink werden dabei vorzugsweise in Form ihrer Sulfate eingesetzt. Kupfer, Kobalt und Eisen werden vorzugsweise in Form von Chelaten, z. B. mit EDTA, eingesetzt. Bor wird vorzugsweise als Natriumborat oder Borsäure eingesetzt. Molybdän wird vorzugsweise als Natrium- oder Ammoniummolybdat oder als Mischung davon eingesetzt. Bezüglich der Mengenanteile der Verbindung der Formel I und den Mikronährstoffen gilt das zuvor Gesagte.

Wie bereits erwähnt, können durch Auflösen der erfindungsgemäßen Zusammensetzungen in einem Lösungsmittel, beispielsweise Wasser, Lösungen der erfindungsgemäßen Zusammensetzungen hergestellt werden. Auf diese Weise können die erfindungsgemäßen Zusammensetzungen unter anderem in Flüssigdüngern zur Bewässerungsdüngung (Fertigation) verwendet werden. Wasser, das von den Wurzeln der Pflanzenkulturen nicht aufgenommen kann, da es z. B. im Substrat oder in für Wurzel nicht erreichbare Bodenschichten versickert, wird als Überschusswasser bezeichnet. Die Durchführung der künstlichen Bewässerungsdüngung mit Flüssigdüngung der erfindungsgemäßen Zusammensetzung kann so erfolgen, dass im Wesentlichen kein Überschusswasser anfällt. Bevorzugt wird die Bewässerungsdüngung mit Flüssigdünger der erfindungsgemäßen Zusammensetzung, insbesondere ein Gemisch aus Flüssigdünger der erfindungsgemäßen Zusammensetzung mit weiteren Additiven, z. B. Ureaseinhibitoren, so durchgeführt, dass kein Überschusswasser anfällt.

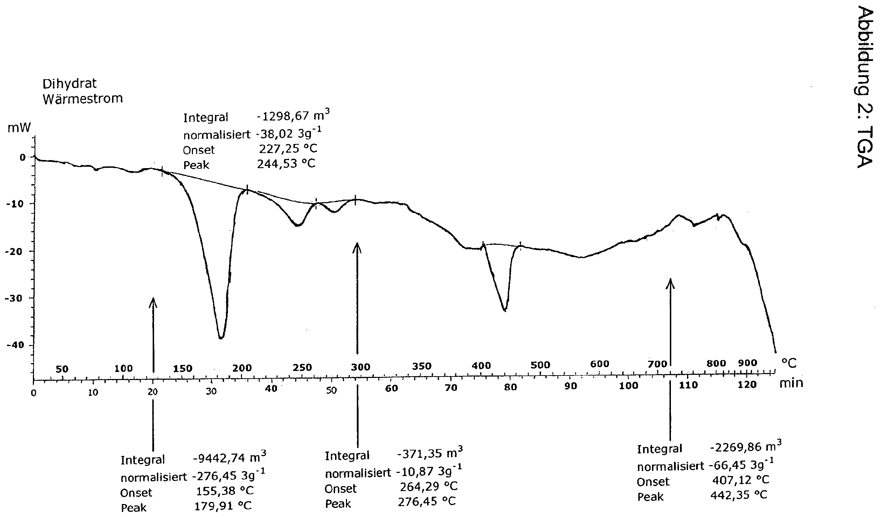

Die erfindungsgemäße Zusammensetzung und die erfindungsgemäßen Verfahren werden durch die nachfolgenden Beispiele und der Abbildungen 1 und Abbildung 2 sowie Tabelle 4 näher erläutert.
Abbildung 1: Zusammenstellung der charakteristischen Reflexe (als d-Werte in Angström angegeben, bestimmt bei 298 K unter Verwendung von Cu-K_{α}-Strahlung: _{α} = 1.5413 Å) und deren relative Intensität im Röntgenpulverdiffraktogramm von der Verbindung der Formel (I) (MgSO₄ • CO(NH₂)₂ • 2 H₂O) und der Verbindung der Formel (II) (MgSO₄ • CO(NH₂)₂ • 3 H₂O). Angaben: d in Angström (1 Å = 0,1 nm), relative Reflexhöhe ist in Bezug auf den 100 % Reflex (≥ 3 %) angegeben
Abbildung 2: Thermogravimetrisches Diagramm der Verbindung der Formel (I).
Abbildung 3: Thermogravimetrisches Diagramm der Verbindung der Formel (II).
Abbildung 4: FTIR-Spektrum der Verbindung der Formel (I).
Abbildung 5: FTIR-Spektrum der Verbindung der Formel (II).

Die Aufnahme der Röntgenpulverdiffraktogramme erfolgte mit einem Typ D 8 Advance Diffraktometer der Fa. Bruker, AXS (298 K, Cu-K_{α}-Strahlung: _{α} = 1.5413 Å), Schrittweite: 0,018385738, Schrittdauer: 0,2 Sekunden, Detektor: Lynx Eye.

Die Elementaranalysen erfolgten durch:
N-Best. DIN ISO 13878, TOC-Best., DIN EN 1484, Mg / S Verbandsmethode LUFA (K+S 0905.01), H2O-Best. Karl-Fischer-Titration.

Die thermogravimetrischen Analysen erfolgten unter Verwendung eines thermogravimetrischen Analysegerätes TGA/DSC 1 der Fa. Mettler-Toledo.

Die IR-Analysen erfolgten mittels eines üblichen FTIR-Spektrometers im Wellenzahlenbereich von 4000 bis 600 cm⁻¹ nach der Methode der abgeschwächten Totalreflexion (ATR).

### Beispiel 1

1000 g (8,31 mol) gemahlenes kalziniertes MgSO₄ (wasserfrei, Korngröße < 0,1 mm, hergestellt aus natürlichem Kieserit) wurden mit 499 g (8,31 mol) gemahlenem Harnstoff (Korngröße < 1,0 mm) vermischt. Hierzu gab man innerhalb von 10 Sekunden 450 g (24,98 mol) Wasser unter intensivem Rühren. Unter Erwärmung auf etwa 87 °C wurde die anfangs flüssige Suspension allmählich teigartig und innerhalb von 5 Minuten zunehmend fester, bis sie sich nicht mehr mischen/rühren lies. Nach weiteren 12 h Ruhezeit (Reifung) an der Luft wurden ein farbloser Feststoff (MgSO₄ • CO(NH₂)₂ • 2 H₂O) erhalten. Die Auswaage betrug 1781 g (8,22 mol), entsprechend einer Ausbeute von 99 %.

### Beispiel 2

500 g (2,13 mol) MgSO₄ • CO(NH₂)₂ • 3 H₂O wurden als pulverförmige Probe 24 h bei 70 °C in einem Labortrockenschrank getrocknet. Nach der Trocknung wurde ein farbloser Feststoff erhalten. Die Auswaage betrug 452,35 g (2,09 mol) MgSO₄ • CO(NH₂)₂ • 2 H₂O, das entspricht einer Ausbeute von 98 %.

### Elementaranalyse:

Die theoretischen und experimentell bestimmten Werte der Verbindungen MgSO₄ • CO(NH₂)₂ • 2 H₂O und MgSO₄ • CO(NH₂)₂ • 3 H₂O sind in nachfolgender Tabelle 4 dargestellt.

**Tabelle 4:**

| | MgSO₄ • CO(NH₂)₂ • 2 H₂O | | MgSO₄ • CO(NH₂)₂ • 3 H₂O | |
|---|---|---|---|---|
| | theoretisch [%] | gefunden [%] | theoretisch [%] | gefunden [%] |
| Mg | 11,2 | 11,1 | 10,4 | 10,8 |
| SO₄²⁻ | 44,4 | 43,6 | 41,0 | 42,5 |
| C | 5,5 | 5,7 | 5,1 | 5,2 |
| N_{ges.} | 12,9 | 12,8 | 11,9 | 12,2 |
| H₂O | 16,6 | 17,2 | 23,0 | 20,4 |

Darüber hinaus wurde mittels der Methode VDLUFA 11.1, 3.6.1 der Biuret-Gehalt des Produktes MgSO₄ • CO(NH₂)₂ • 2 H₂O ermittelt. Die Werte lagen typischerweise bei verschiedenen Herstellchargen um 0,2 % und immer unter 0,9 %.

### Thermogravimetrische Analyse (TGA):

MgSO₄ • CO(NH₂)₂ • 2 H₂O zeigt im thermogravimetrischen Diagramm (Abbildung 2) ab einer Temperatur von ca. 130 °C einen Masseverlust von rund 12 %. Weitere Masseverluste sind ab ca. 205 °C bis 260 °C von 7 % und ab ca. 260 °C bis ca. 290 °C von 3 % zu beobachten. Bei einer Temperatur von 750 °C liegt im Wesentlichen wasserfreies Magnesiumsulfat vor. Die Residualmasse von experimentell rund 52 % (55,6 % theor.) der Ausgangsmasse steht damit in guter Übereinstimmung. Das thermogravimetrische Diagramm ist in Abbildung 2 dargestellt.

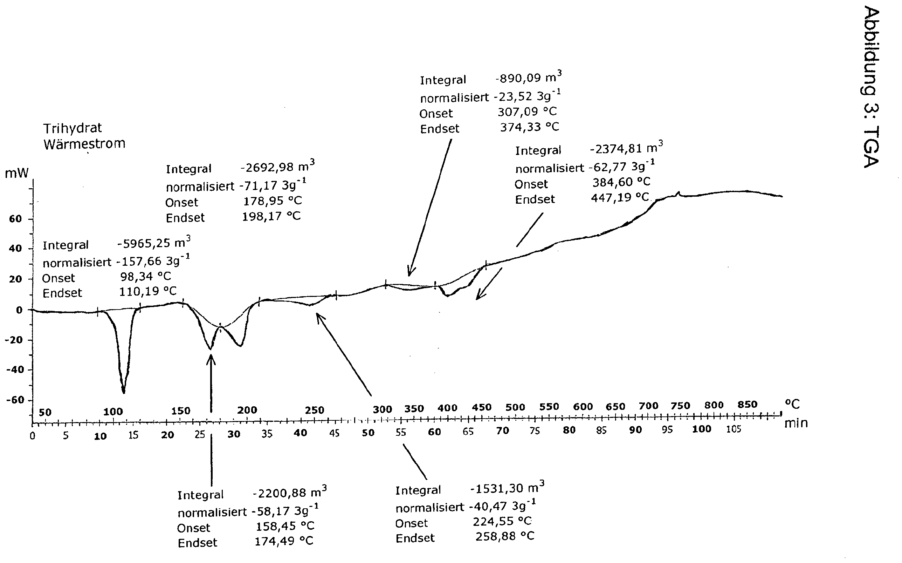

MgSO₄ • CO(NH₂)₂ • 3 H₂O zeigt im thermogravimetrischen Diagramm (Abbildung 3) eine Zersetzung ab einer Temperatur von ca. 90 °C unter einem Masseverlust von ca. 6 %. Ein weiterer Masseverlust von 11 % ist in zwei Stufen ab ca. 155 °C bis ca. 210 °C zu beobachten. Über 210 °C ist ein steter Masseverlust erkennbar. Bei einer Temperatur von 750 °C liegt im Wesentlichen wasserfreies Magnesiumsulfat vor. Die Residualmasse von experimentell rund 50 % (51,4 % theor.) der Ausgangsmasse steht damit in guter Übereinstimmung. Das thermogravimetrische Diagramm ist in Abbildung 2 dargestellt.

Verwendete Abkürzungen (IR-Spektren): m=mittel, st=stark, sst= sehr stark, sh=Schulter, br=breit.

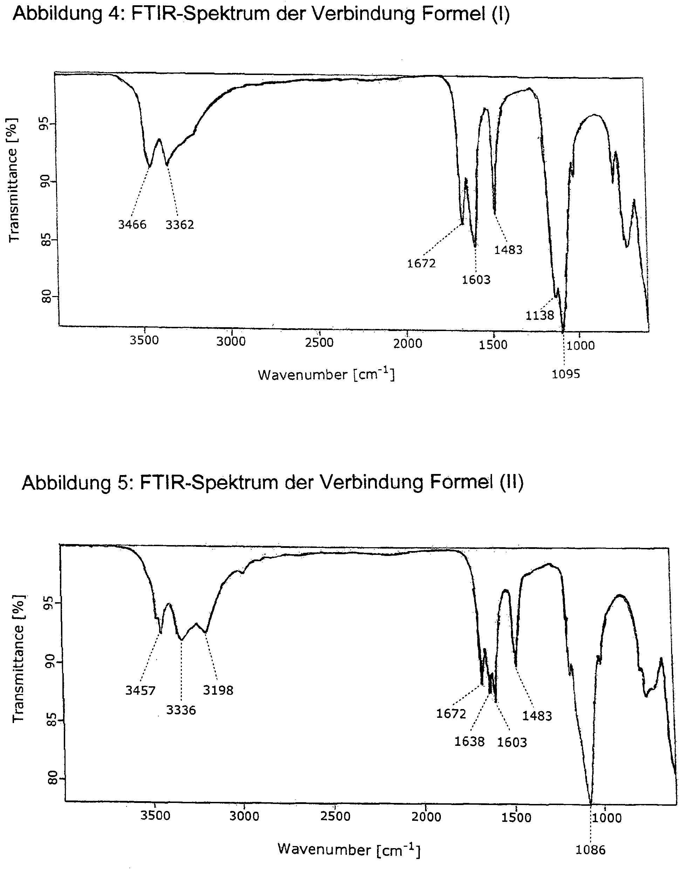

Das IR Spektrum von MgSO₄ • CO(NH₂)₂ • 2 H₂O (Abbildung 4) weist folgende Banden auf: 3466 cm⁻¹ (m, br), 3362 cm⁻¹ (m, br, sh), 1672 cm⁻¹ (st), 1603 cm⁻¹ (st), 1483 cm⁻¹ (st), 1138 cm⁻¹ (sst), 1195 cm⁻¹ (sst).

Das IR Spektrum von MgSO₄ • CO(NH₂)₂ • 3 H₂O (Abbildung 5) weist folgende Banden auf:3457 cm⁻¹ (m, sh), 3336 cm⁻¹ (m, br), 3198 cm⁻¹ (m, br), 1672 cm⁻¹ (st), 1638 cm⁻¹ (st), 1603 cm⁻¹ (st), 1483 cm⁻¹ (st), 1086 cm⁻¹ (sst, br).

## Patentansprüche

1. Zusammensetzung, enthaltend wenigstens 80 Gew.-%, bezogen auf das Gesamtgewicht der Zusammensetzung, einer Magnesiumsulfat-Harnstoff-Verbindung, ausgewählt unter der Verbindung der Formel (I) und Gemischen der Verbindung der Formel (I) mit der Verbindung der Formel (II):
[MgSO₄ • m CO(NH₂)₂ • n H₂O] (I),
[MgSO₄ • x CO(NH₂)₂ • y H₂O] (II),
worin m und x im Bereich von 0,9 bis 1,1, n im Bereich von 1,9 bis < 2,1 und y im Bereich von 2,9 bis 3,1 liegen,
wobei die Zusammensetzung, bezogen auf das Gesamtgewicht der Zusammensetzung, weniger als 10 Gew.-% freies MgSO₄ in Form des Anhydrates oder in Form von Hydraten des Magnesiumsulfats und weniger als 10 Gew.-% ungebundenen Harnstoff enthält, und
wobei der Anteil der Verbindung der Formel (I), bezogen auf die Gesamtmenge an Verbindungen der Formeln (I) und (II), wenigstens 90 Gew.-% beträgt.

2. Zusammensetzung nach Anspruch 1, worin in Formel (I) die Variable m den Wert 1,0 und die Variable n den Wert 2,0 aufweisen und worin in Formel (II) die Variable x den Wert 1,0 und die Variable y den Wert 3,0 aufweisen.

3. Zusammensetzung nach einen der vorherigen Ansprüche, worin der Anteil der Verbindung der Formel (I), bezogen auf die Gesamtmenge an Verbindungen der Formeln (I) und (II), wenigstens 95 Gew.-% beträgt.

4. Verfahren zur Herstellung einer Zusammensetzung, enthaltend wenigstens 80 Gew.-%, bezogen auf das Gesamtgewicht der Zusammensetzung, einer Magnesiumsulfat-Harnstoff-Verbindung, ausgewählt unter der Verbindung der Formel (I) und Gemischen der Verbindung der Formel (I) mit der Verbindung der Formel (II):
[MgSO₄ • m CO(NH₂)₂ • n H₂O] (I),
[MgSO₄ • x CO(NH₂)₂ • y H₂O] (II),
worin m und x im Bereich von 0,9 bis 1,1, n im Bereich von 1,9 bis 2,1 und y im Bereich von 2,9 bis 3,1 liegen, wobei die Zusammensetzung, bezogen auf das Gesamtgewicht der Zusammensetzung, weniger als 10 Gew.-% freies MgSO₄ in Form des Anhydrates oder in Form von Hydraten des Magnesiumsulfats und weniger als 10 Gew.-% ungebundenen Harnstoff enthält,
**dadurch gekennzeichnet, dass** man festes wasserfreies Magnesiumsulfat mit festem Harnstoff und Wasser bei einer Temperatur oberhalb 75°C umsetzt, wobei man wasserfreies Magnesiumsulfat, Harnstoff und Wasser in einem Molverhältnis von Magnesiumsulfat zu Harnstoff im Bereich von 1 : 0,9 bis 1 : 1,1 und Magnesiumsulfat zu Wasser im Bereich von 1 : 2 bis 1 : 4 einsetzt.

5. Verfahren nach Anspruch 4, **dadurch gekennzeichnet, dass** man die Umsetzung in einer Mischvorrichtung mit wenigstens einem rotierenden Mischwerkzeug durchführt und während und/oder unmittelbar im Anschluss an die Umsetzung durch Erhöhung der Drehzahl der Mischwerkzeuge eine Korngrößeneinstellung vornimmt.

6. Verfahren nach einem der Ansprüche 4 oder 5, **dadurch gekennzeichnet, dass** man zu einer Mischung von wasserfreien Magnesiumsulfat und Harnstoff Wasser gibt.

7. Verfahren nach einem der Ansprüche 4 bis 6, **dadurch gekennzeichnet, dass** man das wasserfreie Magnesiumsulfat in Form eines Pulvers einsetzt, worin wenigstens 95 Gew.-% der Partikel einen Durchmesser von maximal 100 µm aufweisen.

8. Verfahren nach einem der Ansprüche 4 bis 7, **dadurch gekennzeichnet, dass** man den Harnstoff in Form eines Pulvers oder Granulats einsetzt, worin wenigstens 95 Gew.-% der Partikel einen Durchmesser von maximal 1000 µm aufweisen.

9. Verfahren zur Herstellung von Zusammensetzungen, enthaltend wenigstens 80 Gew.-%, bezogen auf das Gesamtgewicht der Zusammensetzung, einer Magnesiumsulfat-Harnstoff-Verbindung, ausgewählt unter der Verbindung der Formel (I) und Gemischen der Verbindung der Formel (I) mit der Verbindung der Formel (II):
[MgSO₄ • m CO(NH₂)₂ • n H₂O] (I),
[MgSO₄ • x CO(NH₂)₂ • y H₂O] (II),
worin m und x im Bereich von 0,9 bis 1,1, n im Bereich von 1,9 bis 2,1 und y im Bereich von 2,9 bis 3,1 liegen, wobei die Zusammensetzung, bezogen auf das Gesamtgewicht der Zusammensetzung, weniger als 10 Gew.-% freies MgSO₄ in Form des Anhydrates oder in Form von Hydraten des Magnesiumsulfats und weniger als 10 Gew.-% ungebundenen Harnstoff enthält,
**dadurch gekennzeichnet, dass** man eine Zusammensetzung, die wenigstens 80 Gew.-%, bezogen auf das Gesamtgewicht der Zusammensetzung, der Magnesiumsulfat-Harnstoff-Verbindung der Formel II und weniger als 10 Gew.-% freies MgSO₄ in Form des Anhydrates oder in Form von Hydraten des Magnesiumsulfats und/oder weniger als 10 Gew.-% ungebundenen Harnstoff enthält, bei Temperaturen im Bereich von 65 bis 95° C trocknet.

10. Verwendung einer Zusammensetzung nach einem der Ansprüche 1 bis 3 als Düngemittel oder als Zusatz in Düngemitteln.

11. Düngemittelzusammensetzung in Form einer Stickstoff-Magnesium-Schwefel Düngemittelzusammensetzung, enthaltend eine Zusammensetzung nach einem der Ansprüche 1 bis 3 und Bittersalz.

12. Düngemittelzusammensetzung, enthaltend eine Zusammensetzung nach einem der Ansprüche 1 bis 3 und wenigstens einen weiteren Bestandteil, der unter Ureaseinhibitoren und Nitrifikationsinhibitoren ausgewählt ist.

13. Verfahren zur Bewässerungsdüngung von landwirtschaftlichen oder gärtnerisch genutzten Substraten, bei dem man Wasser, welches eine Zusammensetzung gemäß einem der Ansprüche 1 bis 3 oder eine Düngemittelzusammensetzung nach einem der Ansprüche 11 oder 12 und gegebenenfalls ein oder mehrere weitere Düngemitteladditive so auf ein Substrat einbringt und/oder aufbringt, dass im Wesentlichen kein Überschusswasser anfällt.

## Claims

1. A composition comprising at least 80% by weight, based on the total weight of the composition, of a magnesium sulfate-urea compound selected from the compound of formula (I) and mixtures of the compound of formula (I) with the compound of formula (II):
[MgSO₄ • m CO(NH₂)₂ • n H₂O] (I),
[MgSO₄ • x CO(NH₂)₂ • y H₂O] (II),
where m and x are in the range from 0.9 to 1.1, n is in the range from 1.9 to < 2.1 and y is in the range from 2.9 to 3.1,
wherein the composition, based on the total weight of the composition, contains less than 10% by weight of free MgSO₄ in the form of the anhydrate or in the form of hydrates of magnesium sulfate and less than 10% by weight of unbound urea, and
wherein the proportion of the compound of formula (I) based on the total amount of compounds of formulae (I) and (II) is at least 90% by weight.

2. The composition according to claim 1, wherein in formula (I) the variable m has the value 1.0 and the variable n has the value 2.0, and wherein in formula (II) the variable x has the value 1.0 and the variable y has the value 3.0.

3. The composition according to any of the proceeding claims, wherein the proportion of the compound of formula (I), based on the total amount of compounds of formulae (I) and (II) is at least 95% by weight.

4. A process for producing a composition, comprising at least 80% by weight, based on the total weight of the composition, of a magnesium sulfate-urea compound selected from the compound of formula (I) and mixtures of the compound of formula (I) with the compound of formula (II):
[MgSO₄ • m CO(NH₂)₂ • n H₂O] (I),
[MgSO₄ • x CO(NH₂)₂ • y H₂O] (II),
where m and x are in the range from 0.9 to 1.1, n is in the range from 1.9 to 2.1 and y is in the range from 2.9 to 3.1,
wherein the composition, based on the total weight of the composition, contains less than 10% by weight of free MgSO₄ in the form of the anhydrate or in the form of hydrates of magnesium sulfate and less than 10% by weight of unbound urea,
**characterized in that** solid anhydrous magnesium sulfate is reacted with solid urea and water at a temperature above 75°C, where anhydrous magnesium sulfate, urea and water are used in a mole ratio of magnesium sulfate to urea in the range of 1:0.9 to 1:1.1 and of magnesium sulfate to water in the range of 1:2 to 1:4.

5. The process according to claim 4, **characterized in that** the reaction is carried out in a mixing apparatus having at least one rotating mixing implement and **in that** adjustment of the grain sizes is achieved during and/or directly after the reaction by increasing the speed of the mixing implements.

6. The process according to any of claims 4 or 5, **characterized in that** water is added to a mixture of anhydrous magnesium sulfate and urea.

7. The process according to any of claims 4 to 6, **characterized in that** the anhydrous magnesium sulfate is used in the form of a powder, wherein at least 95% by weight of the particles have a diameter of at most 100 µm.

8. The process according to any of claims 4 to 7, **characterized in that** the urea is used in the form of a powder or granules, wherein at least 95% by weight of the particles have a diameter of at most 1000 µm.

9. A process for producing compositions, comprising at least 80% by weight, based on the total weight of the composition, of a magnesium sulfate-urea compound selected from the compound of formula (I) and mixtures of the compound of formula (I) with the compound of formula (II):
[MgSO₄ • m CO(NH₂)₂ • n H₂O] (I),
[MgSO₄ • x CO(NH₂)₂ • y H₂O] (II),
where m and x are in the range from 0.9 to 1.1, n is in the range from 1.9 to 2.1 and y is in the range from 2.9 to 3.1,
wherein the composition, based on the total weight of the composition, contains less than 10% by weight of free MgSO₄ in the form of the anhydrate or in the form of hydrates of magnesium sulfate and less than 10% by weight of unbound urea,
**characterized in that** a composition comprising at least 80% by weight, based on the total weight of the composition, of the magnesium sulfate-urea compound of formula II and less than 10% by weight of free MgSO₄ in the form of the anhydrate or in the form of hydrates of the magnesium sulfate and/or less than 10% by weight of unbound urea, is dried at temperatures in the range from 65 to 95°C.

10. The use of a composition according to any of claims 1 to 3 as a fertilizer or as an additive in fertilizers.

11. A fertilizer composition in the form of a nitrogen-magnesium-sulfur fertilizer composition, containing a composition according to any of claims 1 to 3 and Epsom salt.

12. A fertilizer composition, containing a composition according to any of claims 1 to 3 and at least one further component selected from urease inhibitors and nitrification inhibitors.

13. A process for fertigation of substrates used in agriculture or horticulture, wherein water containing a composition according to any of claims 1 to 3 or a fertilizer composition according to any of claims 11 or 12 and, optionally, one or more further fertilizer additives is introduced and/or applied to a substrate in such a way that essentially no excess water is produced.

## Revendications

1. Composition, contenant au moins 80 % en poids, par rapport au poids total de la composition, d'un composé de sulfate de magnésium-urée, choisi parmi le composé de formule (I) et les mélanges du composé de formule (I) avec le composé de formule (II) :
[MgSO₄ • m CO(NH₂)₂ • n H₂O] (I),
[MgSO₄ • x CO(NH₂)₂ • y H₂O] (II),
m et x se situant dans la plage allant de 0,9 à 1,1, n se situant dans la plage allant de 1,9 à < 2,1, et y se situant dans la plage allant de 2,9 à 3,1,
la composition contenant, par rapport au poids total de la composition, moins de 10 % en poids de MgSO₄ libre sous la forme de l'anhydrate ou sous la forme d'hydrates de sulfate de magnésium, et moins de 10 % en poids d'urée non liée, et
la proportion du composé de formule (I), par rapport à la quantité totale de composés de formule (I) et (II), étant d'au moins 90 % en poids.

2. Composition selon la revendication 1, dans laquelle, dans la formule (I), la variable m présente la valeur 1,0 et la variable n présente la valeur 2,0, et dans laquelle, dans la formule (II), la variable x présente la valeur 1,0 et la variable y présente la valeur 3,0.

3. Composition selon l'une quelconque des revendications précédentes, dans laquelle la proportion du composé de formule (I), par rapport à la quantité totale de composés de formule (I) et (II), est d'au moins 95 % en poids.

4. Procédé de fabrication d'une composition, contenant au moins 80 % en poids, par rapport au poids total de la composition, d'un composé de sulfate de magnésium-urée, choisi parmi le composé de formule (I) et les mélanges du composé de formule (I) avec le composé de formule (II) :
[MgSO₄ • m CO(NH₂)₂ • n H₂O] (I),
[MgSO₄ • x CO(NH₂)₂ • y H₂O] (II),
m et x se situant dans la plage allant de 0,9 à 1,1, n se situant dans la plage allant de 1,9 à 2,1, et y se situant dans la plage allant de 2,9 à 3,1,
la composition contenant, par rapport au poids total de la composition, moins de 10 % en poids de MgSO₄ libre sous la forme de l'anhydrate ou sous la forme d'hydrates de sulfate de magnésium, et moins de 10 % en poids d'urée non liée,
**caractérisé en ce que** du sulfate de magnésium anhydre solide est mis en réaction avec de l'urée solide et de l'eau à une température supérieure à 75 °C, le sulfate de magnésium anhydre, l'urée et l'eau étant utilisés en un rapport molaire entre le sulfate de magnésium et l'urée dans la plage allant de 1:0,9 à 1:1,1 et entre le sulfate de magnésium et l'eau dans la plage allant de 1:2 à 1:4.

5. Procédé selon la revendication 4, **caractérisé en ce que** la réaction est réalisée dans un dispositif de mélange contenant au moins un outil de mélange rotatif, et un ajustement de la taille des particules est réalisé pendant et/ou directement après la réaction par élévation de la vitesse de rotation des outils de mélange.

6. Procédé selon l'une quelconque des revendications 4 ou 5, **caractérisé en ce que** de l'eau est ajoutée à un mélange de sulfate de magnésium anhydre et d'urée.

7. Procédé selon l'une quelconque des revendications 4 à 6, **caractérisé en ce que** le sulfate de magnésium anhydre est utilisé sous la forme d'une poudre, dans laquelle au moins 95 % en poids des particules présentent un diamètre d'au plus 100 µm.

8. Procédé selon l'une quelconque des revendications 4 à 7, **caractérisé en ce que** l'urée est utilisée sous la forme d'une poudre ou d'un granulat, dans laquelle ou dans lequel au moins 95 % en poids des particules présentent un diamètre d'au plus 1 000 µm.

9. Procédé de fabrication de compositions contenant au moins 80 % en poids, par rapport au poids total de la composition, d'un composé de sulfate de magnésium-urée, choisi parmi le composé de formule (I) et les mélanges du composé de formule (I) avec le composé de formule (II) :
[MgSO₄ • m CO(NH₂)₂ • n H₂O] (I),
[MgSO₄ • x CO(NH₂)₂ • y H₂O] (II),
m et x se situant dans la plage allant de 0,9 à 1,1, n se situant dans la plage allant de 1,9 à 2,1, et y se situant dans la plage allant de 2,9 à 3,1,
la composition contenant, par rapport au poids total de la composition, moins de 10 % en poids de MgSO₄ libre sous la forme de l'anhydrate ou sous la forme d'hydrates de sulfate de magnésium, et moins de 10 % en poids d'urée non liée,
**caractérisé en ce qu'**une composition qui contient au moins 80 % en poids, par rapport au poids total de la composition, du composé de sulfate de magnésium-urée de formule II et moins de 10 % en poids de MgSO₄ libre sous la forme de l'anhydrate ou sous la forme d'hydrates du sulfate de magnésium, et/ou moins de 10 % en poids d'urée non liée, est séchée à des températures dans la plage allant de 65 à 95 °C.

10. Utilisation d'une composition selon l'une quelconque des revendications 1 à 3 en tant qu'engrais ou en tant qu'additif dans des engrais.

11. Composition d'engrais sous la forme d'une composition d'engrais azote-magnésium-soufre, contenant une composition selon l'une quelconque des revendications 1 à 3 et du sel amer.

12. Composition d'engrais, contenant une composition selon l'une quelconque des revendications 1 à 3 et au moins un constituant supplémentaire, qui est choisi parmi les inhibiteurs d'uréase et les inhibiteurs de nitrification.

13. Procédé de fertilisation par irrigation de substrats utilisés en agriculture ou en horticulture, selon lequel de l'eau qui contient une composition selon l'une quelconque des revendications 1 à 3 ou une composition d'engrais selon l'une quelconque des revendications 11 ou 12 et éventuellement un ou plusieurs additifs d'engrais supplémentaires est introduite et/ou appliquée sur un substrat de telle sorte qu'essentiellement aucune eau excédentaire ne soit produite.
